# EUROPÄISCHE PATENTSCHRIFT

(11) **EP 1 391 192 B1**
(45) Veröffentlichungstag und Bekanntmachung des Hinweises auf die Patenterteilung: **18.07.2007**
(21) Anmeldenummer: 02017995.8
(22) Anmeldetag: 12.08.2002
(51) Int. Cl.: A61K 8/04, A61K 8/36, A61K 8/34, A61K 8/39, A61Q 1/02, A61Q 17/04, A61Q 19/00

(54) **System enthaltend eine nachschäumende kosmetische Zubereitung**
System containing a cosmetic post-foaming preparation
Système comprenant une composition cosmétiques post-moussantes

(43) Veröffentlichungstag der Anmeldung: 25.02.2004
(73) Patentinhaber: Beiersdorf Aktiengesellschaft, D-20253 Hamburg (DE)
(72) Erfinder: Kröpke, Rainer, 22869 Schenefeld (DE); Bleckmann, Andreas, 22926 Ahrensburg (DE); Riedel, Heidi, 22529 Hamburg (DE); Rohde, Olaf, 25335 Elmshorn (DE); Trau, Jens, Dr., 22844 Norderstedt (DE)

(56) Entgegenhaltungen:
- EP-A- 0 793 955
- EP-A- 1 216 683
- EP-A- 1 216 684
- WO-A-01/78661
- US-A- 3 970 584

## Beschreibung

Die vorliegende Erfindung betrifft ein System aus schäumbare kosmetische und dermatologische Zubereitungen, insbesondere nachschäumende kosmetische und dermatologische Zubereitungen und einem Behältnis.

Schäume bzw. schaumförmige Zubereitungen gehören zu den dispersen Systemen.

Das bei weitem wichtigste und bekannteste disperse System stellen Emulsionen dar. Emulsionen sind Zwei- oder Mehrphasensysteme von zwei oder mehr ineinander nicht oder nur wenig löslichen Flüssigkeiten. Die Flüssigkeiten (rein oder als Lösungen) liegen in einer Emulsion in einer mehr oder weniger feinen Verteilung vor, die im allgemeinen nur begrenzt stabil ist.

Schäume sind Gebilde aus gasgefüllten, kugel- oder polyederförmigen Zellen, welche durch flüssige, halbflüssige, hochviskose oder feste Zellstege begrenzt werden. Die Zellstege, verbunden über sogenannte Knotenpunkte, bilden ein zusammenhängendes Gerüst. Zwischen den Zellstegen spannen sich die Schaumlamellen (geschlossenzelliger Schaum). Werden die Schaumlamellen zerstört oder fließen sie am Ende der Schaumbildung in die Zellstege zurück, erhält man einen offenzelligen Schaum. Auch Schäume sind thermodynamisch instabil, da durch Verkleinerung der Oberfläche Oberflächenenergie gewonnen werden kann. Die Stabilität und damit die Existenz eines Schaums ist somit davon abhängig, wieweit es gelingt, seine Selbstzerstörung zu verhindern.

Kosmetische Schäume sind in der Regel dispergierte Systeme aus Flüssigkeiten und Gasen, wobei die Flüssigkeit das Dispergiermittel und das Gas die dispergierte Substanz darstellen. Schäume aus niedrigviskosen Flüssigkeiten werden temporär durch oberflächenaktive Substanzen (Tenside, Schaumstabilisatoren) stabilisiert. Solche Tensidschäume haben aufgrund ihrer großen inneren Oberfläche ein starkes Adsorptionsvermögen, welches beispielsweise bei Reinigungs- und Waschvorgängen ausgenutzt wird. Dementsprechend finden kosmetische Schäume insbesondere in den Bereichen der Reinigung, beispielsweise als Rasierschaum, und der Haarpflege Verwendung.

Zur Erzeugung von Schaum wird Gas in geeignete Flüssigkeiten eingeblasen, oder man erreicht die Schaumbildung durch heftiges Schlagen, Schütteln, Verspritzen oder Rühren der Flüssigkeit in der betreffenden Gasatmosphäre, vorausgesetzt, daß die Flüssigkeiten geeignete Tenside oder andere grenzflächenaktive Stoffe (sogenannte Schaumbildner) enthalten, die außer Grenzflächenaktivität auch ein gewisses Filmbildungsvermögen besitzen.

Kosmetische Schäume haben gegenüber anderen kosmetischen Zubereitungen den Vorteil, daß sie eine feine Verteilung von Wirkstoffen auf der Haut erlauben. Allerdings sind kosmetische Schäume in der Regel nur durch Verwendung besonderer Tenside, welche darüber hinaus oft wenig hautverträglich sind, zu erreichen.

Ein weiterer Nachteil des Standes der Technik ist es, daß derartige Schäume nur wenig stabil sind, weshalb sie üblicherweise innerhalb von etwa 24 Stunden zusammenfallen. Eine Anforderung an kosmetische Zubereitungen ist aber, daß diese eine möglichst jahrelange Stabilität besitzen. Diesem Problem wird im allgemeinen dadurch Rechnung getragen, daß der Verbraucher den eigentlichen Schaum erst bei der Anwendung mit Hilfe eines geeigneten Sprühsystems selbst erzeugt, wozu beispielsweise Sprühdosen verwendet werden können, in denen ein verflüssigtes Druckgas als Treibgas dient. Beim Öffnen des Druckventils entweicht das Treibmittel-Flüssigkeitsgemisch durch eine feine Düse, das Treibmittel verdampft und hinterlässt einen Schaum.

Auch nachschäumende kosmetische Zubereitungen sind an sich bekannt. Sie werden zunächst in fließförmiger Form aus einem Aerosolbehälter auf die Haut aufgetragen und entwickeln nach kurzer Verzögerung erst dort unter dem Einfluss des enthaltenen Nachschäummittels den eigentlichen Schaum, beispielsweise einen Rasierschaum. Nachschäumende Zubereitungen liegen oft in speziellen Ausführungsformen wie etwa nachschäumenden Rasiergelen oder dergleichen vor.

Die US-PS 3,541,581 nennt als wesentliche Bestandteile nachschäumender Zubereitungen Wasser, Seife (also wasserlösliche Salze höherer Fettsäuren), Gelstrukturbildner und Nachschäummittel. Auch andere derartige Zubereitungen sind bekannt, die aber alle den Nachteil haben, kosmetisch unelegant zu sein und/oder die Anforderung an niedriges Reizpotential nicht zu erfüllen.

Auch selbstschäumende Duschzubereitungen sind an sich bekannt. So beschreibt die WO 97/03646 Reinigungszubereitungen, welche eine Grundformulierung aus mindestens einem Detergenz und einem Verdicker enthalten, wobei die Viskosität der Grundformulierung größer als 9.500 cps ist. Da durch Zugabe des Treibmittels zur Grundformulierung die Viskosität des Endprodukts stark herabgesetzt wird, haben diese Formulierungen eine hohe Anfangsviskosität, um eine ausreichend hohe Viskosität des Endproduktes (mit Treibmittel) zu erreichen.

Die Zubereitungen des Standes der Technik enthalten dementsprechend üblicherweise hohe Mengen an Verdickungsmitteln. Dabei ist es insbesondere nachteilig, daß sich derartige viskose Zubereitungen nur aufwendig herstellen lassen und zudem in der Produktion Schwierigkeiten bereiten, da man sie nur langsam abfüllen kann.

Aufgabe der vorliegenden Aufgabe war es daher, den Stand der Technik zu bereichern und seinen Nachteilen abzuhelfen.

Es war überraschend und für den Fachmann nicht vorauszusehen, daß ein System enthaltend ein Behältnis und eine Selbstschäumende kosmetische oder dermatologische Zubereitungen, welche aus
I. einem Emulgatorsystem, welches aus
   A. mindestens einem Emulgator A, gewählt aus der Gruppe der ganz-,teil- oder nicht neutralisierten, verzweigten und/oder unverzweigten, gesättigten und/oder ungesättigten Fettsäuren mit einer Kettenlänge von 10 bis 40 Kohlenstoffatomen,
   B. mindestens einem Emulgator B, gewählt aus der Gruppe der polyethoxylierten Fettsäurester mit einer Kettenlänge von 10 bis 40 Kohlenstoffatomen und mit einem E-thoxylierungsgrad von 5 bis 100 und
   C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,
   besteht,
II. bis zu 50 Gew.-%- bezogen auf das Gesamtgewicht der selbstschäumenden Zubereitung - einer Lipidphase, welche ein oder mehrere Lipide enthält,
III. mindestens einem Sekundärtreibmittel, gewählt aus der Gruppe der aliphatischen Kohlenwasserstoffe, wie zum Beispiel n-Pentan, Isopentan und Isobutan, bestehen,
dadurch gekennzeichnet, dass das Behältnis zwei kammern aufweist, wobei die eine kammer die Zubereitung enthält und die zweite kammer ein Primärtreibmittel enthält, so daß die Zubereitung beim Öffnen der ersten Kammer in Freiheit gesetzt wird den Nachteilen des Standes der Technik abhelfen werden.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 1 bis 20 Gew.%, vorteilhaft von 2 bis 15 Gew.%, insbesondere von 5 bis 10 Gew.%, jeweils bezogen auf das Gesamtgewicht der Formulierung, zu wählen.

Es ist erfindungsgemäß vorteilhaft, wenn die Zugabe des Sekundärtreibmittels bei oder vor der Abfüllung der Zubereitung in das Verkaufsbehältnis erfolgt. Dies ist insbesondere deswegen vorteilhaft, da bei Zusatz des Sekundärtreibmittels eine Viskositätserniedrigung (Verdünnung) der Anfangsformulierung erfolgt. Wählt man die Abfüllung des Endproduktes so, daß Anfangsformulierung und Sekundärtreibmittel gemeinsam abgefüllt werden und daß dabei eine intensive Durchmischung erfolgt, so lassen sich sehr hohe Abfüllgeschwindigkeiten erreichen, da die Mischung eine niedrige Viskosität hat.

Unter "selbstschäumend"-ist im Sinne der vorliegenden Erfindung zu verstehen, daß die erfindungsgemäß eingesetzten Zubereitungen Schäume bilden, wenn sie nach dem Verlassen des druckfesten Behälters selbsttätig aufgeschäumt werden, d. h. wenn ein in ihnen enthaltenes - meist gelöstestes - Gas expandiert. In derartig erzeugten Schäumen können die Gasbläschen (beliebig) verteilt in einer (oder mehreren) flüssigen Phase(n) vorliegen, wobei die Schäume makroskopisch nicht notwendigerweise das Aussehen eines Schaumes haben müssen.

Aus erfindungsgemäß eingesetzten selbstschäumenden kosmetischen oder dermatologischen Zubereitungen werden durch Aufschäumen z. B. makroskopisch sichtbar dispergierte Systeme aus in Flüssigkeiten dispergierten Gasen hergestellt werden. Der Schaumcharakter kann aber beispielsweise auch erst unter einem (Licht-) Mikroskop sichtbar werden. Darüber hinaus ist das Aufschäumen der erfindungsgemäßen selbstschäumenden Zubereitungen - insbesondere dann, wenn die Gasbläschen zu klein sind, um unter einem Lichtmikroskop erkannt zu werden - auch an einer starken Volumenzunahme des Systems erkennbar.

Die erfindungsgemäß eingesetzten Zubereitungen stellen in jeglicher Hinsicht überaus befriedigende Präparate dar. Es war insbesondere überraschend, daß die aus den erfindungsgemäßen selbstschäumenden Zubereitungen hergestellten Schäume - auch bei einem ungewöhnlich hohen Gasvolumen - außerordentlich stabil sind. Dementsprechend eignen sich Zubereitungen im Sinne der vorliegenden Erfindung ganz besonders, um als Grundlage für Produktformen mit vielfältigen Anwendungszwecken zu dienen.

Die erfindungsgemäß eingesetzten Zubereitungen und daraus erhältliche Schäume zeigen sehr gute sensorische Eigenschaften, wie beispielsweise die Verteilbarkeit auf der Haut oder das Einzugsvermögen in die Haut, und zeichnen sich darüber hinaus durch eine überdurchschnittlich gute Hautpflege aus.

Aus erfindungsgemäß eingesetzten Zusammensetzungen sind feinblasige, reichhaltige Schäume von hervorragender kosmetischer Eleganz erhältlich. Weiterhin sind aus erfindungsgemäßen Zusammensetzungen besonders gut hautverträgliche Zubereitungen erhältlich, wobei wertvolle Inhaltsstoffe besonders gut auf der Haut verteilt werden können.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate, Behensäure und Behenate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Butyloctanol, Butyldecanol, Hexyloctanol, Hexyldecanol, Octyldodecanol, Behenylalkohol (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist gegebenenfalls vorteilhaft, wenngleich nicht notwendig, wenn die Formulierungen gemäß der vorliegenden Erfindung weitere Emulgatoren enthalten. Vorzugsweise sind solche Emulgatoren zu verwenden, welche zur Herstellung von W/O-Emulsionen geeignet sind, wobei diese sowohl einzeln als auch in beliebigen Kombinationen miteinander vorliegen können.

Vorteilhaft werden der oder die weiteren Emulgatoren aus der Gruppe gewählt, die die folgenden Verbindungen umfasst:
Polyglyceryl-2-Dipolyhydroxystearat, PEG-30-Dipolyhydroxystearat, Cetyldimethiconcopolyol, Glykoldistearat, Glykoldilaurat, Diethylenglykoldilaurat, Sorbitantrioleat, Glykololeat, Glyceryldilaurat, Sorbitantristearat, Propylenglykolstearat, Propylenglykollaurat, Propylenglykoldistearat, Sucrosedistearat, PEG-3 Castor Oil, Pentaerythritylmonostearat, Pentaerythritylsesquioleat, Glyceryloleat, Glycerylstearat, Glyceryldiisostearat, Pentaerythritylmonooleat, Sorbitansesquioleat, Isostearyldiglycerylsuccinat, Glycerylcaprat, Palm Glycerides, Cholesterol, Lanolin, Glyceryloleat (mit 40 % Monoester), Polyglyceryl-2-Sesquiisostearat, Polyglyceryl-2-Sesquioleat, PEG-20 Sorbitan Beeswax, Sorbitanoleat, Sorbitanisostearat, Trioleylphosphat, Glyceryl Stearate und Ceteareth-20 (Teginacid von Th. Goldschmidt), Sorbitanstearat, PEG-7 Hydrogenated Castor Oil, PEG-5-Soyasterol, PEG-6 Sorbitan Beeswax, Glycerylstearat SE, Methylglucosesesquistearate, PEG-10 Hydrogenated Castor Oil, Sorbitanpalmitat, PEG-22/Dodecylglykol Copolymer, Polyglyceryl-2-PEG-4-Stearat, Sorbitanlaurat, PEG-4-Laurat, Polysorbat 61, Polysorbat 81, Polysorbat 65, Polysorbat 80, Triceteareth-4-Phosphat, Triceteareth-4 Phosphate und Sodium C₁₄₋₁₇ Alkyl Sec Sulfonat (Hostacerin CG von Hoechst), Glycerylstearat und
PEG-100 Stearate (Arlacel 165 von ICI), Polysorbat 85, Trilaureth-4-Phosphat, PEG-35 Castor Oil, Sucrosestearat, Trioleth-8-Phosphat, C₁₂₋₁₅ Pareth-12, PEG-40 Hydrogenated Castor Oil, PEG-16 Soya Sterol, Polysorbat 80, Polysorbat 20, Polyglyceryl-3-methylglucose Distearat, PEG-40 Castor Oil, Natriumcetearylsulfat, Lecithin, Laureth-4-Phosphat, Propylenglykolstearat SE, PEG-25 Hydrogenated Castor Oil, PEG-54 Hydrogenated Castor Oil, Glycerylstearat SE, PEG-6 Caprylic/Capric Glycerides, Glyceryloleat und Propylenglykol, Glyceryllanolat, Polysorbat 60, Glycerylmyristat, Glycerylisostearat und Polyglyceryl-3 Oleat, Glyceryllaurat, PEG-40-Sorbitanperoleat, Laureth-4, Glycerinmonostearat, Isostearylglycerylether, Cetearyl Alcohol und Natriumcetearylsulfat, PEG-22-Dodecylglykolcopolymer, Polyglyceryl-2-PEG-4-Stearat, Pentaerythrithylisostearat, Polyglyceryl-3-Diisostearat, Sorbitanoleat und Hydrogenated Castor Oil und Cera alba und Stearinsäure, Natriumdihydroxycetylphosphat und Isopropylhydroxycetylether, Methylglucosesesquistearat, Methylglucosedioleat, Sorbitanoleat und PEG-2 Hydrogenated Castor Oil und Ozokerit und Hydrogenated Castor Oil, PEG-2 Hydrogenated Castor Oil, PEG-45-/Dodecylglykolcopolymer, Methoxy PEG-22-/Dodecylglykolcopolymer, Hydrogenated Coco Glycerides, Polyglyceryl-4-Isostearat, PEG-40-Sorbitanperoleat, PEG-40-Sorbitanperisostearat, PEG-8-Beeswax, Laurylmethiconcopolyol, Polyglyceryl-2-Laurat, Stearamidopropyl-PG-dimoniumchloridphosphat, PEG-7 Hydrogenated Castor Oil, Triethylcitrat, Glycerylstearatcitrat, Cetylphosphat, Polyglycerolmethylglucosedistearat, Poloxamer 101, Kaliumcetylphosphat, Glycerylisostearat, Polyglyceryl-3-Diisostearate.

Bevorzugt werden der oder die weiteren Emulgatoren im Sinne der vorliegenden Erfindung aus der Gruppe der hydrophilen Emulgatoren gewählt. Erfindungsgemäß besonders bevorzugt sind Mono-, Di-, Trifettsäureester der Sorbitane.

Die Gesamtmenge der weiteren Emulgatoren wird erfindungsgemäß vorteilhaft kleiner als 5 Gew.-%, bezogen auf das Gesamtgewicht der Formulierung, gewählt.

Die Liste der genannten weiteren Emulgatoren, die im Sinne der vorliegenden Erfindung eingesetzt werden können, soll selbstverständlich nicht limitierend sein.

Besonders vorteilhafte selbstschäumende und/oder schäumbare Zubereitungen im Sinne der vorliegenden Erfindung sind frei von Mono- oder Diglycerylfettsäureestern. Insbesondere bevorzugt sind erfindungsgemäße Zubereitungen, welche kein Glycerylstearat, Glycerylisostearat, Glyceryldiisostearat, Glyceryloleat, Glycerylpalmitat, Glycerylmyristat, Glyceryllanolat und/oder Glyceryllaurat enthalten.

Die schäumbaren kosmetischen oder dermatologischen Mittel gemäß der Erfindung können beispielsweise aus Zweikammeraerosolbehältern entnommen und auf die Haut aufgetragen werden. Erfindungsgemäße Packmittel sind Behältnisse, in der sich eine Kammer mit einer Füllung aus den flüssigen bzw. breiartigen Stoffen, unter dem Druck eines in einer zweiten Kammer befindlichen stehenden Primärtreibmittels befindet. Derartige Behälter können mit Ventilen sehr unterschiedlicher Bauart ausgestattet sein, die die Entnahme des Inhalts der ersten Kammer als Emulsion oder Gel in jeder Lage - auch mit dem Ventil nach unten - ermöglichen.
Eine besondere Ausführungsform sind BiCan^{®}-Aerosolbehälter, bei denen das Produkt in einem flexiblen Beutel aus Metall oder Kunststoff innerhalb der Dose eingeschlossen ist.

Erfindungsgemäß eingesetzte Zusammensetzungen stellen ungeschäumt, also unmittelbar nach dem Austreten aus dem Aerosolbehälter, Zwei- oder Mehrphasensysteme - in der Regel Emulsionen - dar. Sie können bereits durch leichtes Verreiben, beispielsweise in den Händen oder beim Auftragen und Verreiben auf der Haut, aber auch durch Rühren oder sonstige Aufschäumvorgänge zu Schäumen gestaltet werden.

Es hat sich darüber hinaus in überraschender Weise herausgestellt, daß bei der Verwendung von Treibmitteln, besonders vorteilhaft von in der gegebenenfalls vorhandenen Ölphase löslichen Treibmitteln, also beispielsweise üblichen Propan-Butan-Gemischen, die erfindungsgemäßen Zubereitungen nicht einfach als Aerosoltröpfchen versprüht werden, sondern sich zu feinblasigen, reichhaltigen Schäumen entwickeln, sobald solche mit solchen Treibmitteln beladenen Systeme Druckentspannung erfahren.

Bei Verwendung von Kohlenwasserstoffen oder deren Gemischen mit 4 oder 5 Kohlenstoffatomen, insbesondere Isobutan, n-Pentan und Isopentan, kann man das selbständige Aufschäumen nach dem Austritt aus der Druckverpackung zeitlich verzögern.

Durch das Verdampfen des Sekundärtreibmittels im applizierten Kosmetikprodukt wird der Haut Wärme entzogen und ein angenehmer Kühleffekt erlangt.

Solche nachschäumenden Zubereitungen werden daher ebenfalls als vorteilhafte Verkörperungen der vorliegenden Erfindung mit eigenständiger erfinderischer Tätigkeit angesehen.

Erfindungsgemäß sind feinblasige, reiche Schäume von hervorragender kosmetischer Eleganz erhältlich. Weiterhin sind erfindungsgemäß besonders gut hautverträgliche Zubereitungen erhältlich, wobei wertvolle Inhaltsstoffe besonders gut auf der Haut verteilt werden können.

Die erfindungsgemäßen kosmetischen und/oder dermatologischen Zubereitungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Eine weitere vorteilhafte Ausführungsform der vorliegenden Erfindung besteht in After-Sun-Produkten, insbesondere als Pflegeschäume mit kühlender Wirkung.

Erfindungsgemäß können schaumförmige oder schäumbare kosmetische und dermatologische Zubereitungen auch als Reinigungsmittel eingesetzt werden.

Auch als Rasiermittel, beispielsweise Rasierschäume, aber auch sonstigen Pre- und Aftershave-Zubereitungen können die erfindungsgemäßen Zubereitungen Anwendung finden.

Erfindungsgemäße kosmetische Zubereitungen, die ein Hautreinigungsmittel oder Shampoonierungsmittel darstellen, enthalten vorzugsweise zusätzlich mindestens eine anionische, nicht-ionische oder amphotere oberflächenaktive Substanz, oder auch Gemische aus solchen Substanzen und Hilfsmittel, wie sie üblicherweise dafür verwendet werden. Die oberflächenaktive Substanz bzw. die Gemische aus diesen Substanzen können in einer Konzentration zwischen 1 Gew.-% und 94 Gew.-%, insbesondere bis zu 50 Gew.-% in dem Shampoonierungsmittel vorliegen.

Erfindungsgemäße kosmetische Zubereitungen können nach Abbau (Zerfall) der Schaumstruktur die Form einer Lotion,
- die nicht ausgespült wird, insbesondere zum Einlegen der Haare oder
- die beim Fönen der Haare verwendet wird,
entsprechen. Ein solcher Frisier- und Behandlungsschaum, stellt im allgemeinen eine wässrige, alkoholische oder wässrig-alkoholische Lösung dar und enthält gegebenenfalls zusätzlich mindestens ein kationisches, anionisches, nicht-ionisches oder amphoteres Polymer oder auch Gemische derselben, sowie erfindungsgemäße Emulgatorkombinationen in wirksamer Konzentration. Die Menge der verwendeten Polymeren liegt z.B. zwischen 0,1 und 10 Gew.-%, bevorzugt zwischen 0,1 und 3 Gew.-%.

Erfindungsgemäß eingesetzte kosmetische Zubereitungen können zur Behandlung und Pflege der Haut und Haare neben einem wirksamen Gehalt an erfindungsgemäßen Wirkstoffkombinationen und dafür üblicherweise verwendeten Lösungsmitteln, bevorzugt Wasser, noch Gelbildner, z.B. organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdckkungsmittel, z.B. Aluminiumsilikate wie beispielsweise Bentonite, oder ein Gemisch aus Polyethylenglykol und Polyethylenglycolstearat oder - distearat, enthalten. Das Verdickungsmittel ist in der Zubereitung z.B. in einer Menge zwischen 0,1 und 30 Gew.-%, bevorzugt zwischen 0,5 und 15 Gew.-%, enthalten.

Die erfindungsgemäß eingesetzte Zusammensetzungen können ferner gegebenenfalls in der Kosmetik übliche Zusatzstoffe, beispielsweise Parfüm, Verdicker, Desodorantien, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate enthalten.

Besonders vorteilhaft ist ein Zusatz von öllöslichen UV-Filtern und/oder UV-Strahlung absorbierender bzw. reflektierender anorganischer Pigmente.

Vorteilhafte öllösliche UVB-Filtersubstanzen sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte öllösliche UVA-Filtersubstanzen sind z.B.:
- Derivate des Dibenzoylmethans, vorzugsweise 1-(4'-tert.Butylphenyl)-3-(4'-methoxyphenyl)propan-1,3-dion
- 1-Phenyl-3-(4'-isopropylphenyl)propan-1,3-dion.

Erfindungsgemäß eingesetzte kosmetische und dermatologische Zubereitungen enthalten außerdem vorteilhaft, aber nicht zwingend, anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃, Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich um Pigmente auf der Basis von TiO₂, BaSO₄.

Die anorganischen Pigmente liegen bevorzugt in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

n TiO₂ + m (RO)₃Si-R' -> (TiO₂)ₙ₋ₘ[TiO(OR)(Si(OR')₃]ₘ

erzeugt wird n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen T 805 von der Firma Degussa oder MT 100 T von der Firma Tayca oder M 160 von der Frima Kemira erhältlich.

Als wasserdispergierbare anorganische Mikropigmente können beispielsweise solche Produkte gewählt werden, welche unter der Handelsbezeichnung Tioveil® von der Firma Tioxide erhältlich sind.

Die Gesamtmenge an anorganischen Pigmenten, insbesondere hydrophoben anorganischen Mikropigmenten (als an sich fakultativ einzusetzender zusätzlicher Substanz) in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Ölphase der erfindungsgemäßen Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und lsotridecylisononanoat, aus Cyclomethicon und 2-Ethylhexylisostearat.

Vorteilhaft beträgt der Gehalt an der Ölphase zwischen 1 und 50 Gew.-%, bezogen auf das Gesamtgewicht der Zubereitungen, bevorzugt 2,5 - 30 Gew.-%, insbesondere bevorzugt 5 - 15 Gew.-%.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, 1,2-Propandiol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1 (schaumförmige O/W-Creme):

| **Emulsion I** | **Gew.-%** |
|---|---|
| Stearinsäure | 3,00 |
| Cetylalkohol | 8,50 |
| PEG-20 Stearat¹ | 8,50 |
| C₁₂₋₁₅ Alkylbenzoat² | 4,00 |
| Paraffinöl³ | 5,00 |
| Isohexadecan⁴ | 2,00 |
| Glycerin | 5,00 |
| Natriumhydroxid | q.s. |
| Konservierung | q.s. |
| Parfum | q.s. |
| Wasser,demineralisiert | ad 100 |
| pH-Wert eingestellt auf 6,5-8,5 | |
| Zur Herstellung des Schaums werden 95 Gew.-% der Emulsion 1 mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |

| | |
|---|---|
| ¹Myrj 49, ICI Surfactants, ² Finsolv TN, WITCO Goldschmidt, ³ Pionier 2071, DEA Mineralöl, ⁴ Solvent ICH, EC Erdölchemie Bayer AG | |

### Beispiel 2 (O/W-Lotion):

| **Emulsion II** | **Gew.-%** |
|---|---|
| Stearinsäure | 2,00 |
| Myristylalkohol | 1,50 |
| Cetylstearylalkohol | 0,50 |
| PEG-100 Stearat¹ | 3,0 |
| Mineralöl ² | 5,00 |
| Hydriertes Polyisobuten ³ | 15,0 |
| Cyclomethicon ⁴ | 5,00 |
| Glycerin | 8,00 |
| Natriumhydroxid | q.s. |
| Konservierung | q.s. |
| Parfum | q.s. |
| Wasser,demineralisiert | ad 100 |
| pH-Wert eingestellt auf 5,0-6,5 | |
| Zur Herstellung des Schaums werden 95 Gew.-% der Emulsion II mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |
| ¹ Myrj 59p, ICI Surfactants, ² Hydrobrite 1000 PO, Witco BV, ³ Polysynlan, Chemische Fabrik Lehrte, ⁴ Dow Corning Fluid 245, Dow Corning | |

### Beispiel 3 (O/W-Lotion):

| **Emulsion III** | **Gew.-%** |
|---|---|
| Stearinsäure | 5,00 |
| Cetylstearylalkohol | 5,50 |
| PEG-30 Stearat¹ | 1,00 |
| Cyclomethicon ² | 3,00 |
| Isoeikosan ³ | 10,00 |
| Octyldodecanol ⁴ | 10,00 |
| Citronensäure | 0,10 |
| Glycerin | 3,00 |
| Parfüm, Konservierungsmittel, | q.s. |
| Natriumhydroxid | q.s. |
| Farbstoffe usw. | q.s. |
| Wasser | ad 100 |
| pH-Wert eingestellt auf 6,0-8,5 | |
| Zur Herstellung des Schaums werden 95 Gew.-% der Emulsion III mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |

| | |
|---|---|
| ¹ Myrj 51, ICI Surfactants, ² Dow Corning Fluid 245, Dow Corning, ³ Isoeikosan, EC Erdölchemie GmbH, ⁴ Eutanol G, Cognis AG | |

### Beispiel 4 (O/W-Emulsions-Make-up):

| **Emulsion IV** | **Gew.-%** |
|---|---|
| Palmitinsäure | 2,00 |
| Cetylalkohol | 2,00 |
| PEG-100 Stearat¹ | 2,00 |
| Dimethicon ² | 2,50 |
| Paraffinöl ³ | 9,50 |
| Dicaprylylcarbonat ⁴ | 2,00 |
| Glycerin | 3,00 |
| Glimmer | 1,00 |
| Eisenoxid | 1,00 |
| Titandioxid | 4,50 |
| Retinylpalmitat | 0,10 |
| Natriumhydroxid | q.s. |
| Konservierung | q.s. |
| Parfum | q.s. |
| Wasser,demineralisiert | ad 100 |
| pH-Wert eingestellt auf 6,0-8,5 | |
| Zur Herstellung des Schaums werden 95 Gew.-% der Emulsion IV mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |

| | |
|---|---|
| ¹ Myrj 59p, ICI Surfactants, ² Wacker Silikonöl AK 35, Wacker, ³Pionier 6301, DEA Mineralöl, ⁴ Cetiol OE, Cognis AG | |

### Beispiel 5 (O/W-Creme):

| **Emulsion V** | **Gew.-%** |
|---|---|
| Stearinsäure | 4,00 |
| Cetylalkohol | 2,00 |
| PEG-30 Stearat¹ | 2,00 |
| Sorbitanmonostearat ² | 1,50 |
| Paraffinöl ³ | 5,00 |
| Cyclomethicon⁴ | 5,00 |
| Vitamin E Acetate | 1,00 |
| Retinylpalmitat | 0,20 |
| Glycerin | 3,00 |
| BHT | 0,02 |
| Disodium EDTA | 0,10 |
| Parfüm, Konservierungsmittel, Farbstoffe | q.s. |
| Kaliumhydroxid | q.s. |
| Wasser | ad 100 |
| pH-Wert eingestellt auf 5,0-8,5 | |
| Zur Herstellung des Schaums werden 95 Gew.-% der Emulsion V mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |

| | |
|---|---|
| ¹ Myrj 51, ICI Surfactants, ² Glycomol S, Akzo Nobel, ³ Pionier 2076, DEA Mineralöl ⁴ Dow Corning Fluid 245, Dow Corning | |

### Beispiel 6 (O/W-Lotion):

| **Emulsion VI** | **Gew.-%** |
|---|---|
| Stearinsäure | 4,00 |
| Cetylstearylalkohol | 1,00 |
| PEG-100 Stearat¹ | 1,00 |
| Paraffin Öl² | 6,50 |
| Dimethicon ³ | 2,50 |
| Vitamin E Acetat | 2,00 |
| Glycerin | 20,00 |
| Parfüm, Konservierungsmittel, Farbstoffe | q.s. |
| usw. | |
| Natriumhydroxid | q.s. |
| Wasser | ad 100 |
| pH-Wert eingestellt auf 6,0-7,5 | |
| Zur Herstellung des Schaums werden 95 Gew.-% der Emulsion VI mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |

| | |
|---|---|
| ¹Myrj 59p, ICI Surfactants, ² Pionier 2076, DEA Mineralöl, ³ Wacker Silikonöl AK 50, Wacker | |

### Beispiel 8 (schaumförmige O/W-Creme):

| **Emulsion I** | **Gew.-%** |
|---|---|
| Stearinsäure | 3,00 |
| Cetylalkohol | 8,50 |
| PEG-20 Stearat¹ | 8,50 |
| C₁₂₋₁₅ Alkylbenzoat² | 4,00 |
| Paraffinöl³ | 5,00 |
| Isohexadecan⁴ | 2,00 |
| Glycerin | 5,00 |
| Triethanolamin | q.s. |
| Konservierung | q.s. |
| Parfum | q.s. |
| Wasser,demineralisiert | ad 100 |
| pH-Wert eingestellt auf 6,5-8,5 | |
| Zur Herstellung des Schaums werden 95 Gew.-% der Emulsion I mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |
| ¹ Myrj 49, ICI Surfactants, ² Finsolv TN, Finetex, ³ Pionier 2071, DEA Mineralöl, ⁴ Solvent ICH, EC Erdölchemie Bayer AG | |

### Beispiel 9 (O/W-Lotion) :

| **Emulsion** | **Gew.-%** |
|---|---|
| Stearinsäure | 2,00 |
| Behenylalkohol | 1,50 |
| Cetylstearylalkohol | 0,50 |
| PEG-100 Stearat ¹ | 3,0 |
| Mineralöl ² | 5,00 |
| Isopropylpalmitat | 15,0 |
| Cyclomethicon ³ | 5,00 |
| Glycerin | 8,00 |
| Natriumhydroxid | q.s. |
| Konservierung | q.s. |
| Parfum | q.s. |
| Wasser,demineralisiert | ad 100 |
| pH-Wert eingestellt auf 5,0-6,5 | |
| Zur Herstellung des Schaums werden 95 Gew.-% der Emulsion mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |
| ¹ Myrj 59p, ICI Surfactants, ² Hydrobrite 1000 PO, Witco BV, ³ Dow Corning Fluid 245, Dow Corning | |

### Beispiel 10 (O/W-Lotion):

| **Emulsion** | **Gew.-%** |
|---|---|
| Stearinsäure | 5,00 |
| Cetylstearylalkohol | 5,50 |
| PEG-30 Stearat¹ | 1,00 |
| Cyclomethicon ² | 12,00 |
| Octyldodecanol ³ | 10,00 |
| Citronensäure | 0,10 |
| Glycerin | 15,00 |
| Parfüm, Konservierungsmittel, | q.s. |
| Natriumhydroxid | q.s. |
| Farbstoffe usw. | q.s. |
| Wasser | ad 100 |
| pH-Wert eingestellt auf 6,0-8,5 Zur Herstellung des Schaums werden 95 Gew.-% der Emulsion mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |

| | |
|---|---|
| ¹ Myrj 51, ICI Surfactants, ² Dow Corning Fluid 245, Dow Corning, ³ Eutanol G, Cognis AG | |

### Beispiel 11 (O/W-Make-up-Emulsion):

| **Emulsion** | **Gew.-%** |
|---|---|
| Behensäure | 2,00 |
| Cetylalkohol | 2,00 |
| PEG-100 Stearat ¹ | 2,00 |
| Dimethicon ² | 2,50 |
| Paraffinöl ³ | 2,50 |
| Dicaprylylcarbonat ⁴ | 9,00 |
| Glycerin | 8,00 |
| Glimmer | 1,00 |
| Eisenoxid | 1,00 |
| Titandioxid | 4,50 |
| Retinylpalmitat | 0,10 |
| Natriumhydroxid | q.s. |
| Konservierung | q.s. |
| Parfum | q.s. |
| Wasser,demineralisiert | ad 100 |
| pH-Wert eingestellt auf 6,0 -8,5 | |
| Zur Herstellung des Schaums werden 95 Gew.-% der Emulsion mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |

| | |
|---|---|
| ¹ Myrj 59p, ICI Surfactants, ² Wacker Silikonöl AK 35, Wacker, ³ Pionier 6301, DEA Mineralöl, ⁴ Cetiol OE, Cognis AG | |

### Beispiel 12 (OIW-Creme):

| **Emulsion** | **Gew.-%** |
|---|---|
| Stearinsäure | 4,00 |
| Cetylalkohol | 2,00 |
| PEG-30 Stearat ¹ | 2,00 |
| Sorbitanmonostearat ² | 1,50 |
| Paraffinöl ³ | 5,00 |
| Cyclomethicon ⁴ | 5,00 |
| Vitamin E Acetate | 1,00 |
| Retinylpalmitat | 0,20 |
| Sorbitol | 3,00 |
| BHT | 0,02 |
| Disodium EDTA | 0,10 |
| Parfüm, Konservierungsmittel, Farbstoffe | q.s. |
| Kaliumhydroxid | q.s. |
| Wasser | ad 100 |
| pH-Wert eingestellt auf 5,0-8,5 | |
| Zur Herstellung des Schaums werden 95 Gew.-% der Emulsion mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |

| | |
|---|---|
| ¹ Myrj 51, ICI Surfactants, ²Glycomol S, Akzo Nobel, ³Pionier 2076, DEA Mineralöl ⁴ Dow Corning Fluid 245, Dow Corning | |

### Beispiel 13 (O/W-Lotion) :

| **Emulsion** | **Gew.-%** |
|---|---|
| Stearinsäure | 4,00 |
| Cetylstearylalkohol | 1,00 |
| PEG-100 Stearat¹ | 1,00 |
| Paraffin Öl ² | 6,50 |
| Isopropylstearat ³ | 2,50 |
| Vitamin E Acetat | 2,00 |
| Butylenglykol | 20,00 |
| Parfüm, Konservierungsmittel, Farbstoffe | q.s. |
| usw. | |
| Natriumhydroxid | q.s. |
| Wasser | ad 100 |
| pH-Wert eingestellt auf 6,0-7,5 Zur Herstellung des Schaums werden 95 Gew.-% der Emulsion mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |
| ¹ Myrj 59p, ICI Surfactants, ² Pionier 2076, DEA Mineralöl, ³ Isopropylstearat, Cognis AG | |

### Beispiel 14 (Sonnenschutz-Lotion):

| **Emulsion** | **Gew.-%** |
|---|---|
| Stearinsäure | 1,00 |
| Cetylstearylalkohol | 4,00 |
| Myristylalkohol | 1,00 |
| PEG-20 Stearat¹ | 1,00 |
| Caprylsäure/Caprinsäuretriglycerid ² | 12,00 |
| C12-15 Alkylbenzoat ³ | 5,50 |
| Dimethicon ⁴ | 0,50 |
| Octyl Isostearat ⁵ | 5,00 |
| Glycerin | 10,00 |
| Octylmethoxycinnamat ⁶ | 4,00 |
| Butylmethoxydibenzoylmethan ⁷ | 3,00 |
| Ethylhexyltriazon ⁸ | 3,00 |
| BHT | 0,02 |
| Disodium EDTA | 0,10 |
| Parfüm, Konservierungsmittel, Farbstoffe, | q.s. |
| usw. | |
| Kaliumhydroxid | q.s |
| Wasser | ad 100 |
| pH-Wert eingestellt auf 5,0-6,0 | |
| Zur Herstellung des Schaums werden 95 Gew.-% der Emulsion mit 5 Gew.-% eines Sekundärtreibmittel (z.B. 75% Isopentan und 25% Isobutan) eingesetzt und mit dem Primärtreibmittel (z.B. Druckluft, Propan/Butan-Mischung) aufgeschäumt. | |

| | |
|---|---|
| ¹ Myrj 49, ICI Surfactants, ² Miglyol 812, Uniqema, ³ Finsolv TN, Finetex, ⁴ Wacker Silikonöl AK 50, Wacker, ⁵ Prisorine 2036, Uniqema, ⁶ Escalol 5571, ISP-Van Dyke, ⁷ Parsol 1789, Hoffmann La Roche, ⁸ Uvinul T150 | |

## Patentansprüche

1. System enthaltend ein Behältnis und eine Selbstschäumende kosmetische oder dermatologische Zubereitungen, welche aus
I. einem Emulgatorsystem, welches aus
A. mindestens einem Emulgator A, gewählt aus der Gruppe der ganz- teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,
besteht,
II. bis zu 50 Gew.-% - bezogen auf das Gesamtgewicht der selbstschäumenden Zubereitung - einer Lipidphase, welche ein oder mehrere Lipide enthält
III. mindestens einem Sekundärtreibmittel, gewählt aus der Gruppe der aliphatischen Kohlenwasserstoffe, wie zum Beispiel n-Pentan, Isopentan und Isobutan, bestehen,
**dadurch gekennzeichnet, dass** das Behältnis zwei kammern aufweist wobei die eine Kammer die Zubereitung enthält und die zweite Kammer ein Primärtreibmittel enthält, so daß die Zubereitung bein Öffnen des ersten Kammer in Freiheit gesetzt wird

2. System nach Anspruch 1, **dadurch gekennzeichnet**, das die Gewichtsverhältnisse von Emulgator A zu Emulgator B zu Coemulgator- C (A : B : C) in der Zubereitung wie a : b : c gewählt werden, wobei a, b und c unabhängig voneinander rationale Zahlen von 1 bis 5, bevorzugt von 1 bis 3 darstellen können, insbesondere bevorzugt ist ein Gewichtsverhältnis von etwa 1:1:1.

3. System nach Anspruch 1 oder 2, **dadurch gekennzeichnet**, das die Gesamtmenge der Emulagatoren A und B und des Coemulgators C in der Zubereitung aus dem Bereich von 1 bis 20 Gew.-%, vorteilhaft von 2 bis 15 Gew.-%, insbesondere von 5 bis 10 Gew.-%, jeweils bezogen auf das Gesamtgewicht der Formulierung, zu wählen ist.

4. System nach Anspruch 1 bis 3, **dadurch gekennzeichnet**, das die Zubereitung weitere kosmetische Zusatz- und/oder Wirkstoffe enthalten kann, insbesondere weitere Emulgatoren, deren Gesamtmenge aber kleiner als 5 Gew.-% bezogen auf das Gesamtgewicht ist

5. System nach einem oder mehreren der vorhergehenden Ansprüche bei der durch das Verdampfen des Sekundärtreibmittels im applizierten Kosmetikprodukt der Haut Wärme entzogen wird und ein angenehmer Kühleffekt erlangt wird.

6. Verwendung eines Systems nach einem oder mehreren der vorhergehenden Ansprüche für ein Sonnenschutz mittel, After-Sun-Produkte, Reinigungsmittel oder Rasiermittel.

## Claims

1. System comprising a container and a self-foaming cosmetic or dermatological preparation which consists of
I. an emulsifier system which consists of
A. at least one emulsifier A selected from the group of completely neutralized, partially neutralized or unneutralized branched and/or unbranched, saturated and/or unsaturated fatty acids with a chain length of from 10 to 40 carbon atoms,
B.at least one emulsifier B selected from the group of polyethoxylated fatty acid esters with a chain length of from 10 to 40 carbon atoms and with a degree of ethoxylation of from 5 to 100 and
C.at least one coemulsifier C selected from the group of saturated and/or unsaturated, branched and/or unbranched fatty alcohols with a chain length of from 10 to 40 carbon atoms,
II. up to 50% by weight - based on the total weight of the self-foaming preparation - of a lipid phase which comprises one or more lipids,
III. at least one secondary propellant selected from the group of aliphatic hydrocarbons, such as, for example n-pentane, isopentane and isobutane,
**characterized in that** the container has two chambers, where one chamber comprises the preparation and the second chamber comprises a primary propellant, so that the preparation is set free upon opening the first chamber.

2. System according to Claim 1, **characterized in that** the weight ratios of emulsifier A to emulsifier B to coemulsifier C (A:B:C) in the preparation are chosen as a:b:c, where a, b and c, independently of one another, can be rational numbers from 1 to 5, preferably from 1 to 3, particular preference being given to a weight ratio of about 1:1:1.

3. System according to Claim 1 or 2, **characterized in that** the total amount of emulsifiers A and B and of coemulsifier C in the preparation is to be chosen from the range from 1 to 20% by weight, advantageously from 2 to 15% by weight, in particular from 5 to 10% by weight, in each case based on the total weight of the formulation.

4. System according to Claim 1 to 3, **characterized in that** the preparation can comprise further cosmetic additives and/or active ingredients, in particular further emulsifiers, but whose total amount is less than 5% by weight, based on the total weight.

5. System according to one or more of the preceding claims in which, as a result of the vaporization of the secondary propellant in the applied cosmetic product, heat is withdrawn from the skin and a pleasant cooling effect is achieved.

6. Use of a system according to one or more of the preceding claims for sunscreens, after-sun products, cleansing compositions or shaving compositions.

## Revendications

1. Système contenant un récipient et une préparation cosmétique ou dermatologique auto-moussante, qui se compose
I.d'un système d'émulsifiants, qui consiste en
A. au moins un émulsifiant A, sélectionné parmi le groupe des acides gras saturés et/ou insaturés, ramifiés et/ou non ramifiés, complètement, partiellement ou non neutralisés, ayant une longueur de chaîne de 10 à 40 atomes de carbone,
B.Au moins un émulsifiant B, sélectionné parmi le groupe des esters d'acide gras polyéthoxylés, ayant une longueur de chaîne de 10 à 40 atomes de carbone et ayant un degré d'éthoxylation allant de 5 à 100, et
C.au moins un co-émulsifiant C, sélectionné parmi le groupe des alcools gras ramifiés et/ou non ramifiés, saturés et/ou insaturés, ayant une longueur de chaîne de 10 à 40 atomes de carbone,
II. de jusqu'à 50% en poids, par rapport au poids total de la préparation auto-moussante, d'une phase lipide, qui contient un ou plusieurs lipides,
III. d'au moins un agent porogène secondaire, sélectionné parmi le groupe des hydrocarbures aliphatiques, comme, par exemple, le n-pentane, l'isopentane et l'isobutane,
**caractérisé en ce que** le récipient présente deux chambres, la première chambre contenant la préparation et la deuxième chambre contenant un agent porogène primaire, de telle sorte que la préparation soit libérée à l'ouverture de la première chambre.

2. Système selon la revendication 1, **caractérisé en ce que** les rapports pondéraux de l'émulsifiant A à l'émulsifiant B au co-émulsifiant C (A:B:C) dans la préparation sont sélectionnés comme a:b:c, a, b, et c pouvant représenter, indépendamment les uns des autres, des nombres rationnels allant de 1 à 5, de préférence, de 1 à 3, un rapport pondéral d'environ 1:1:1 étant particulièrement préféré.

3. Système selon la revendication 1 ou 2, **caractérisé en ce que** la quantité totale des émulsifiants A et B et du co-émulsifiant C dans la préparation est à choisir dans le domaine de 1 à 20% en poids, avantageusement de 2 à 15% en poids, en particulier de 5 à 10% en poids, à chaque fois par rapport au poids total de la formulation.

4. Système selon la revendication 1 à 3, **caractérisé en ce que** la préparation peut contenir des additifs et des principes actifs cosmétiques supplémentaires, en particulier des émulsifiants supplémentaires, dont la quantité totale est cependant inférieure à 5% en poids, par rapport au poids total.

5. Système selon une ou plusieurs des revendications précédentes, dans lequel l'évaporation de l'agent porogène secondaire dans le produit cosmétique appliqué permet d'éliminer une sensation de chaleur de la peau et d'obtenir un effet rafraîchissant agréable.

6. Utilisation d'un système selon une ou plusieurs des revendications précédentes pour des agents de protection solaire, des produits après-soleil, des agents de nettoyage ou des agents de rasage.
